(19) Europäisches Patentamt
European Patent Office
Office européen des brevets

(11) Publication number: **0 165 963**
**B1**

(12) # EUROPEAN PATENT SPECIFICATION

(45) Date of publication of patent specification: **09.08.89**

(51) Int. Cl.$^4$: **C 12 P 19/04,** C 08 B 37/00

(21) Application number: **85900215.6**

(22) Date of filing: **13.12.84**

(86) International application number:
**PCT/NL84/00042**

(87) International publication number:
**WO 85/02626 20.06.85 Gazette 85/14**

(54) **PROCESS FOR THE RECOVERY OF A GELFORMING POLYSACCHARIDE, THE RECOVERED POLYSACCHARIDE AS WELL AS THE GEL FORMED THEREWITH.**

(30) Priority: **14.12.83 NL 8304304**

(43) Date of publication of application:
**02.01.86 Bulletin 86/01**

(45) Publication of the grant of the patent:
**09.08.89 Bulletin 89/32**

(84) Designated Contracting States:
**AT BE CH DE FR GB LI LU NL SE**

(56) References cited:
**Carbohydrate Research, vol. 124, 29 December 1983, Elsevier Science Publishers B.V. Amsterdam (NL), L.P.T.M. Zevenhuizen et al.: "Gelforming capsular polysaccharide of Rhizobium leguminosarum and Rhizobium trifolii", pp. 166-171**

**Carbohydrate Research, vol. 111, 1983, Elsevier Scientific Publishing Comp., Amsterdam (NL), R. Beyer et al.: "Structural study of the extracellular polysaccharide gum from Rhizobium strain CB744", pp. 195-203**

(73) Proprietor: **COÖPERATIEVE VERENIGING SUIKER UNIE U.A.**
**Zuilenstraat 100**
**NL-4814 ND Breda (NL)**

(72) Inventor: **VAN NEERVEN, Alexander, Raymundus, Wilhelmus**
**Treubstraat 79**
**NL-6702 BB Wageningen (NL)**
Inventor: **ZEVENHUIZEN, Ludovicus, Petrus, Theodorus, Maria**
**Schuurhoven 11**
**NL-6721 SM Bennekom (NL)**

(74) Representative: **van der Beek, George Frans, Ir. et al**
**Nederlandsch Octrooibureau Scheveningseweg 82 P.O. Box 29720**
**NL-2502 LS 's-Gravenhage (NL)**

**EP 0 165 963 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
ANTONIE VAN LEEUWENHOEK, vol. 47, 1981, L.P.T.M. Zevenhuizen: "Cellular glycogen, beta-1,2-glucan, polybeta-hydroxybutyric acid and extracellular polysaccharides in fastgrowing species of Rhizobium", pp. 481-497

Chemical Abstracts, vol. 98, no. 17, 25 April 1983, Columbus, Ohio, (US), R.W. Carlson et al.: "A comparison of the surface polysaccharides from Rhizobium leguminosarum 128C53 smRrifR with the surface polysaccharides from its exo-1 mutant", p. 302, abstract no. 140182j

Chemical Abstracts, vol. 83, no. 21, 24 November 1975, Columbus, Ohio (US), B. Courtois et al.: "Polysaccharides in Rhizobium during rapid growth", p. 273, abstract no. 175211k

Chemical Abstracts, vol. 92, no. 21, 1980, Columbus, Ohio, (US), L.P.T.M. Zevenhuizen et al.: "Surface carbohydrates of Rhizobium. Part II. Lipoplysaccharides of Rhizobium", p. 302, abstract no. 177139c

**Description**

The invention relates to a gel-forming polysaccharide.

As is known, polysaccharide hydrocolloids which are obtained from plants and seaweeds are successfully employed in the foodstuff, petroleum and textile industries as well as in many other industries. The commercial use of these polysaccharides is inter alia based on the ability of these to modify the rheological properties of water as desired. Such polysaccharides are of particular importance in the foodstuff industry and in oil production, especially with regard to methods for increasing the oil yield of drillings.

Moreover, it is known that polysaccharides are extracellularly excreted by many microorganisms. In view of the fact that such microbial polysaccharides possess unique physically and chemically constant properties, and can be obtained on a regular basis, a number of these microbially obtained polysaccharides have proved to be of commercial interest.

As is mentioned above, polysaccharides can be obtained from natural sources (vegetable or microbial) while at the same time synthetic sources are also of note. Examples of natural and synthetic sources are gum arabic, caraya and tragacanth (plant exudates), guar and locust bean gum (seed extracts), agar products, alginates and carrageenan (seaweed extracts), starch (maize and potato), pectin, gelatine, dextran and xanthan gum (natural products), dextrins (starch), carboxymethylcellulose (cellulose), polyvinyl alcohol, polyacrylic acid and ethylene oxide polymers (synthetic products).

In recent years, it is particularly the synthetic and microbially obtained gums which have been greatly in the ascendant at the expense of the vegetable gums. Characteristic uses of such gums, for example as stabilisers and suspending agents, thickeners, film-forming agents, moisture-retaining colloids, coagulants, lubricants and the like are to be found in the area of detergents, textiles, adhesives, paper, paints, feedstuffs, drugs and cosmetics. Moreover, some microbially prepared polysaccharides can, after hydrolysis, also be used as starting materials for the preparation of synthetic oligosaccharides and polysaccharides.

Examples of specific microbially prepared polysaccharides are:

1) Dextran (molecular weight about 100,000) prepared from sucrose under the action of culture filtrates of *leuconostoc mesenteroides.*

2) Xanthan gum, prepared from glucose with the aid of *Xanthomonas capestris* (Kelco Co.).

3) Alginates prepared with the aid of *Azotobacter vinelandii* (Tate and Lyle).

4) Curdlan, prepared with the aid of *Alcaligenes faecalis* (Takeda Co.)

The first two products are characterised by a strong ability to increase the viscosity of aqueous solutions whilst the two last-mentioned products possess a considerable gel-forming capacity in aqueous solutions.

An investigation has shown that varieties of *Agrobacterium* predominantly produce two types of high molecular weight extracellular polysaccharides, namely

(a) succinoglycan, which is soluble in water and is responsible for the high-viscosity behaviour of the cultures and

(b) curdlan, which is insoluble in cold water but is soluble in 0.5M NaOH and hot water. The succinoglycan is built up to glucose, galactose, pyruvate and succinate in a ratio of 7:1:1:1. Curdlan, which is built up of β-(1 → 3)-glucan and is reported to be insoluble in cold water but soluble in 0.5M NaOH and in hot water, gives with water, after neutralisation or cooling respectively, a fairly stiff, resilient but thermo-irreversible gel. Moreover it has been found that the gelling properties of curdlan leave something to be desired, since, in order to obtain a gel, curdlan must be present in a concentration of at least 1% or more, sometimes even 2 to 3%.

In Antonie van Leeuwenhoek (1981) *47*, pages 481—497, especially page 485, second and third paragraph, a process is disclosed for the isolation of cellular glycogen, β-1,2-glucan, poly-β-hydroxybutyric acid (PHB) and extracellular polysaccharides from several species of the genus *Rhizobium* like *R. leguminosarum* RCR 1044 and *R. trifolii* TA—1. More in particular the exopolysaccharides were obtained from the cell-free supernatant of the culture of a *Rhizobium* strain by adding ethanol whereas the glycogen, β-1,2-glucan and the PHB were isolated from the cells by carrying out an ultrasonification stage in water and subsequently an extraction procedure for the recovered cell fragments and the obtained aqueous cell extract respectively. For the sake of completeness it is pointed at page 494, second paragraph of the above Antonie van Leeuwenhoek-reference indicating that cell-bound loose amorphous slime present with many *Rhizobium* strains was extracted with 0.5 N NaOH and reprecipitated by neutralization with HCl. In this way merely a gel-forming β-1,3-glucan, so called Curdlan (vide above) was obtained.

Moreover, it has been found that the gel strength of known agar products such as Gibco agar and Oxoid Ion agar No. 2 is undesirably low for a number of applications, especially if the concentration by weight is low.

It has been found that the abovementioned disadvantages of known products can be overcome by a polysaccharide built up of repeat units having the formula:

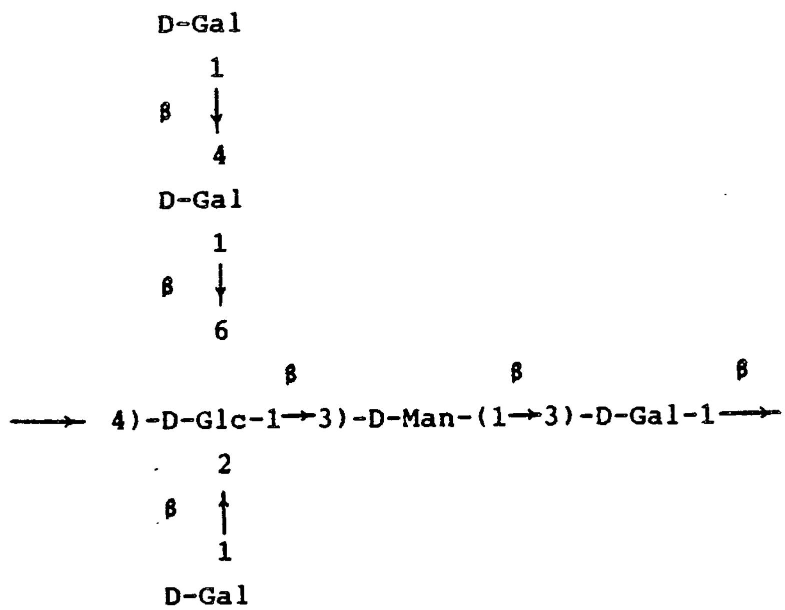

wherein D-Gal, D-Glc and D-Man stand for D-galactose, D-glucose and D-mannose, the polysaccharide having a mean molecular weight of at least 150,000. (The above mean molecular weight has been determined by gel chromatography of the main chain over Ultragel A—4 with dextrans of known molecular weight as reference substance).

In particular, using a polysaccharide according to the invention a stiff gel is obtained even at a weight concentration of only 0.2%, the gel strength of this gel being substantially greater than that of a gel obtained with a known agar such as Gibco agar.

The gelforming polysaccharide according to the invention may be obtained by extraction of cultures of the genus *Rhizobium.* More especially, the varieties *Rhizobium geluminosarum, Rhizobium trifolii* and *Rhizobium phaseoli* of the genus *Rhizobium,* are used. In this respect the invention further relates to a recovery process for the gelforming polysaccharide, defined above according to which the *Rhizobium leguminosarum* strains F—13 and RCR 1044 or *Rhizobium trifolii* strain TA—1 (deposited at the Laboratory for Microbiology in Delft, Holland under Nos. LMD 83.29, LMD 83.28 and LMD 83.30 which depository institution is officially recognized by the Dutch Patent Office) are used.

The extraction of the cultures of the genus *Rhizobium* is carried out, for example, with the aid of an alkaline solution such as an M NaOH solution at room temperature, after which the extract obtained is neutralized with dilute acid. This gives a stiff gel having a high gel strength.

The extraction of the cultures of the genus *Rhizobium* can also be carried out with almost boiling water, after which the suspension obtained is passed through a bacteriological filter. On cooling to room temperature the clear filtrate solidifies to a stable and stiff gel.

As mentioned, a stiff gel according to the invention is obtained even at a concentration of only 0.2% of polysaccharide in water. The gel is thermoreversible, with a transition temperature between the gel and sol state of between 50 and 60°C (solidification point and melting point respectively).

Experimental

The following strains were investigated:

*Rhizobium leguminosarum* F—13, TOM, RCR—1012 and RCR—1044.

*Rhizobium trifolii* TA—1 and 0403 and *Rhizobium phaseoli* 127K—82, all present in the public bacteria collection of the Laboratory for Microbiology in Wageningen.

Carbohydrate analysis of the cultures

2 ml of a culture are pipetted into a centrifuge tube after which 2 ml of 2N NaOH are added. An extraction takes place by shaking for 2 hours at room temperature. Thereafter, the extracted cells, which contain non-extracted glycogen, are centrifuged off and the supernatant liquor, which contains the extracted polysaccharide, is poured into a second centrifuge tube. The capsule polysaccharide (CPS) is precipitated by adding 3 ml of 2N HCl, centrifuged off, decanted and redissolved in 4 ml of 2N NaOH. 0.1 ml thereof is taken for a determination of the carbohydrate content with the aid of the anthrone/sulphuric acid reaction. The supernatant liquor, which contains separated-out acid, exopolysaccharide (EPS) and β-1,2-glucan, can be used further for the determination of these carbohydrate constituents.

The invention is further explained with reference to the example which follows, but is in no way restricted thereto:

Example

Culturing of *Rhizobium trifolii*, strain TA—1, in glutamic acid/lactose/salts medium

Composition of the medium:

| | | | |
|---|---|---|---|
| Glutamic acid | 1 g | C source: lactose 10 g | |
| $K_2HPO_4$ | 1 g | in 100 ml of water | |
| $MgSO_4.7H_2O$ | 0.2 g | | |
| Trace elements: | $FeCl_3.6H_2O$ | 2.5 mg | |
| | $H_3BO_3$ | 0.01 mg | |
| | $ZnSO_4.7H_2O$ | 0.01 mg | |
| | $CoCl_2.6H_2O$ | 0.1 mg | |
| | $CuSO_4.5H_2O$ | 0.01 mg | |
| | $MnCl_2$ | 1 mg | |
| | $Na_2MoO_4.2H_2O$ | 0.01 mg | |
| Vitamins: | biotin | 10 µg | |
| | thiamine | 100 µg | |
| demineralised water | | 1000 ml | |

After having dissolved the constituents in 1 litre of demineralised water, the medium is brought to a pH of 7.0 by adding N NaOH. 50 ml amounts are metered into 200 ml Erlenmeyer flasks; larger quantities of 1 litre are metered into 5 litre Erlenmeyer flasks, then provided with cottonwool plugs and sterilized in an autoclave for 20 minutes at 120°C. The C source, lactose, is sterilised separately and after it has cooled is added aseptically to the base medium.

Culturing is carried out by inoculating a preculture of 50 ml of medium from a sloping tube containing a pure culture of *Rhizobium trifolii* strain TA—1 on *Rhizobium agar* (yeast extract, 0.1%; mannitol, 0.5%; $K_2HPO_4$, 0.05%; $MgSO_4.7H_2O$, 0.025%; $CaCl_2.2H_2O$, 0.01%; agar, 1.0% in water). The culture is incubated on a rotary shaking machine at 25—30° for 5—7 days. This preculture is then used to inoculate 1 litre of the same medium in a 5 litre Erlenmeyer flask and the latter is incubated on a shaking machine at 30° for 7 days.

Isolation of the gel-forming polysaccharide from the Rhizobium culture

After the end of the culture, the bacteria are centrifuged off in a high speed (12,000 rpm) centrifuge. The clear supernatant liquor thereby obtained can optionally be used to isolate the acid exopolysaccharide (EPS) excreted by the bacteria, by precipitation with alcohol or by forming a complex with cetylpyridinium chloride (L.P.T.M. Zevenhuizen. Methylation analysis of acidic exopolysaccharides of *Rhizobium* and *Agrobacterium*. Carbohyd. Res. 26, 409—419 (1973)).

The neutral gel-forming capsule polysaccharide (CPS) is insoluble and is found in the cell pellet obtained. A determination of the quantities of EPS and CPS formed can be carried out in a simple manner by means of a hexose determination using the anthrone/sulphuric acid method; the consumption of the C source (lactose) can be determined by measuring the reducing capacity of the supernatant liquor, using the method of Somogyi-Nelson.

The cell pellet obtained is suspended in 200 ml of water after which 200 ml of 2N NaOH are added. Extraction takes place by stirring for 1—2 hours at room temperature. The extracted cells are subsequently centrifuged off and the clear viscous supernatant liquor is acidified slightly with acetic acid or with dilute hydrochloric acid. Hereupon, a voluminous gel separates out; this is centrifuged off, washed several times in the centrifuge tube, dialysed if appropriate, and freeze-dried. Yield: about 2.3 g of dry polysaccharide material.

After culturing in an 0.1% glutamic acid/0.5% mannitol/salts medium (see J. Microbiol. Serol. 47 (1981), 481—497), the abovementioned strains RCR 1044 and F—13 gave 200—300 mg of product containing more than 95% of hexose (calculated on D-galactose), determined by means of the anthrone/ sulphuric acid method. The abovementioned strains RCR 1012, 0403, TOM, 127 K—82 and various other strains of the genus *Rhizobium* yielded smaller quantities of the same product.

Determination of the structure of the polysaccharide.

TABLE I

Sugar analysis of the original and modified capsule polysaccharide of *Rhizobium trifolii* strain TA—1.

| Sugar component as alditol acetates) | Molar ratio [a] | | | | | |
|---|---|---|---|---|---|---|
| | A | B | C | D | E | F |
| Glycerol | | + | | | | |
| Threitol | | 0.7 | | | | |
| Erythritol | | | | | + | |
| D-Mannose | 1.0 | 1.00 | 1.00 | 1.00 | 1.00 | 0.17 |
| D-Galactose | 4.0 | 0.92 | 1.03 | 1.41 | 0.99 | 0.34 |
| D-Glucose | 1.0 | 0.97 | 0.96 | 0.96 | 0.14 | 0.11 |

[a]Polysaccharide: A, original; B periodate-oxidised and borohydride-reduced; C periodate-oxidised, borohydride-reduced and Smith-cleavage (main chain); D, partially periodate-oxidised (0.5 hour), borohydride-reduced and Smith-cleavage; E, periodate-oxidised and borohydride-reduced (main chain); and F, chromium trioxide-oxidised, acetylated original polysaccharide.

TABLE II

Methylation analysis of the original and modified capsule polysaccharide of *Rhizobium trifolii*, strain TA—1

| Methylated sugar[a] | T[b] | Molar ratio [c] | | | | | |
|---|---|---|---|---|---|---|---|
| | | A | B | C | D | E | F |
| 2,3,4,6-Man | 1.00; | | | | | | 1.00 |
| 2,3,4,6-Gal | 1.07 | 1.80 | | | | 0.34 | |
| 2,4,6-Man | 1.54 | 1.00 | 1.00 | 1.00 | 1.00 | 1.00 | |
| 2,4,6-Gal | 1.58 | 0.96 | 0.99 | 1.05 | 1.11 | 0.93 | 0.83 |
| 2,3,6-Gal | 1.65 | 0.84 | | | | | |
| 2,3,6-Glc | 1.79 | | | 0.88 | 0.83[d] | 0.46 | |
| 2,3-Glc | 2.98 | | | | | 0.70 | |
| 3-Glc | 4.58 | 0.87 | 0.88 | | | | |

[a] 2,3,4,6-Man = 2,3,4,6-tetra-O-methyl-D-mannose, and so on.
[b] Retention time of the corresponding alditol-acetates relative to that of 1,5-di-O-acetyl-2,3,4,6-tetra-O-methyl-D-glucitol on a "fused silica" WCOT—OV—225 column at 190°C.
[c] Polysaccharide: A, original; B, periodate-oxidised and borohydride-reduced; C, periodate-oxidised, borohydride-reduced and Smith-cleavage; D, periodate-oxidised, borohydride-reduced, methylated, Smith-cleavage and trideuterio-methylated; E, partially periodate-oxidised (0.5 hour), borohydride-reduced and Smith-cleavage; F, periodate-oxidised, borohydride-reduced, Smith-cleavage main chain.
[d] 2,6-Di-O-trideuteriomethyl-3-O-methyl-D-glucose.

The hydrolysis of the polysaccharide obtained (2M trifluoroacetic acid, 100°C, 6 hours) gave D-galactose, D-glucose and D-mannose in a molar ratio of 4:1:1 (Table I, column A), which suggests a hexasaccharide repeat unit. No uronic acids, or acyl-, (acetyl-, succinyl-) or acetal-bonded pyruvate substituents were detected. The methylation analysis of the polysaccharide (Table II, column A) shows two D-galactosyl end groups, one (1 → 3)-bonded D-mannosyl residue, one (1 → 3)-bonded D-galactosyl residue and one (1 → 4) D-galactosyl residue, and a doubly-branched D-glucosyl residue, via 0-1,2,4,6, to be present.

The polysaccharide took up five moles of periodate per repeat hexasaccharide unit and gave two moles of formic acid, in the course of 5 hours at room temperature. The borohydride reduction of the polyaldehyde obtained, followed by hydrolysis in 2M trifluoroacetic acid, gave D-glucose, D-galactose and D-mannose in a ratio of 1:1:1 together with glycerol and threitol (Table I, column B). Methylation of the polyalcohol (Table II, column B) indicated that the periodate-oxidation had cleaved two D-galactosyl end groups and one (1 → 4)-bonded D-galactosyl residue. The Smith cleavage of the polyalcohol (90% formic acid, 40°C, 1 hour) gave a product which was still macro-molecular and non-dialysable and contained equal quantities of D-glucose, D-galactose and D-mannose (Table I, column C). A methylation analysis of the Smith-cleaved polymer (Table II, column C) indicated that the latter was unbranched and consisted of (1 → 3)-bonded D-mannosyl residues, (1 → 3)-bonded D-galactosyl residues and (1 → 4)-bonded D-glucosyl residues. The bonding points of the D-galactosyl side chains were fixed by methylation of the polyalcohol, followed by Smith cleavage and trideuteriomethylation of the cleaved polymer (Table II, column D). The 2,3,6-tri-O-methyl-D-glucose obtained was MeO-2,6-deuterated, indicating the positions of the D-galactosyl and the di-D-galactosyl side chains. The relative positions were established by methylation analysis of partially periodate-oxidised and Smith-cleaved polysaccharide (Table I, column D). Since a D-galactosyl end group which possesses a vicinal triol group is considered to be more sensitive to periodate attack than a (1 → 4)-bonded D-galactosyl residue which contains a vicinal diol group, each non-oxidised D-galactosyl residue which remains bonded to the main chain after such an oxidation will indicate the position of the di-D-galactosyl side chain. The formation of 2,3-di-O-methyl-D-glucose via methylation analysis of incompletely periodate-oxidised and Smith-cleaved polysaccharide (Table II, column E) indicated that the di-D-galactosyl side chain occupied position 6 of the main chain-D-glucosyl residue.

The sequence of the hexose residues in the main chain was investigated by periodate oxidation of the polysaccharide stripped of the side chains, since the now (1 → 4)-bonded D-glucosyl residues are sensitive to periodate oxidation (Table I, column E). A successive reduction and Smith cleavage of the polyalcohol gave a trisaccharide product of D-mannose, D-galactose and erythritol, methylation analysis of which (Table II, column F) gave 2,3,4,6-tetra-O-methyl-D-mannose and 2,4,6-tri-O-methyl-D-galactose, which indicated the following sequence: 3)-D-Man-(1 → 3)-D-Gal-(1 → 4)-D-Glc-(1 → in the main chain.

A chromium trioxide oxidation of the acylated original polysaccharide followed by a sugar analysis (Table I, column F) showed that all sugar residues were oxidised and consequently the residues were mainly β-bonded.

From the above data it was possible to deduce the following structure for the gel-forming polysaccharide according to the invention.

```
                  D-Gal
                    1
                  β ↓
                    4
                  D-Gal
                    1
                  β ↓
                    6
                       β              β              β
    ──→ 4)-D-Glc-1──→3)-D-Man-(1──→3)-D-Gal-1──→
                    2
                  β ↑
                    1
                  D-Gal
```

The above structure includes some unusual aspects for a bacterial exopolysaccharide which is build up of repeat units. It is a neutral heteropolysaccharide without carbonyl-containing substituents and therefore

resembles the extracellular polysaccharide gum from the slow-growing *Rhizobium* strain CB 744 of the cowpea group (Arch. Biochem. Biophys. 124 (1968) 292—298). The structure also contains a doubly-branched sugar residue, which is an unusual feature of bacterial polysaccharides. Similar features are also present in the capsule polysaccharides of *Klebsiella*, types 33 and 38 (Carbohydrate Research 70 (1979) 134—144). The above structure of the gel-forming polysaccharide according to the invention is the first to be reported for an insoluble capsule polysaccharide of a fast-growing strain of *Rhizobia*.

Methods of structure determination

The sugar analysis and methylation analysis employed in the structure determination are described in Carbohydrate Research 26 (1973) 409—419 and Chem. Commun. Univ. Stockholm, 8 (1976) 1—75.

The periodate oxidation.

Polysaccharide (100 mg) was dissolved in water (50 ml) at 60°C. 0.03M sodium metaperiodate (50 ml) was added to the stirred solution after which the mixture was immediately cooled to room temperature. Samples (0.1 ml) were diluted at intervals to 25 ml and the periodate consumption was determined spectrophotometrically at 223 nm. The liberation of formic acid was determined by titration with 0.1M NaOH after reduction of unconverted periodate with ethylene glycol. The oxidation took place rapidly in the dark at 20°C; after 8 hours, an excess of ethylene glycol was added and the oxidised product was isolated by dialysis and subsequently reduced overnight with sodium borohydride (200 mg). The excess reducing agent was cleaved with acetic acid and the mixture was dialysed and freeze-dried.

The Smith cleavage.

The Smith cleavage of the product (84.8 mg) was carried out for 1 hour at 40°C in 90% formic acid (40 ml). The formic acid was evaporated off in vacuo and a solution of the residue in water was dialysed and freeze-dried. A portion (33.1 mg) of the product (43.1 mg) was dissolved in 15 mM $NaIO_4$ (40 ml). After 4 days in the dark, the mixture was worked up in the manner described above. A part (10 mg) of the product (30 mg) was subjected to a sugar analysis and another part (10 mg) was subjected for 1 hour at 40°C to a Smith cleavage in 90% formic acid (5 ml). The solution was evaporated in vacuo and freeze-dried, after which the residue was subjected to a methylation analysis.

The chromium trioxide oxidation.

The chromium trioxide oxidation was carried out in the manner described by Lindberg and Lönngren (Methods Enzymol. 50 (1978), 20—24).

Comparison of the gel strengths of the capsule polysaccharide of Rhizobium trifolii TA—1 and of two known agar products

The results of the gel strength determinations are shown in the attached figure.

The gel strengths are determined by measuring the breaking strengths of the gels by means of an Overload Dynamics type S—100 gel tester; the plunger diameter of the load cell was 12.7 mm. The gels to be investigated were prepared by heating, followed by cooling in water at various weight concentrations (0.1—2.0% weight/volume).

It can be deduced from the attached figure that the bacterial gel according to the invention has a higher gel strength, at the same weight concentrations, than two known agars used as comparison material, namely Gibco agar and Oxoid Ion agar No. 2. In particular, at low weight concentrations (0.1—0.4%) fairly stiff gels were still obtained with the bacterial polysaccharide according to the invention, while this was hardly any longer possible with the two agars in the said concentration range. Further, the bacterial product proves to be more resilient than the known agars, in that the bacterial gel permitted a greater deformation (8 mm penetration depth of the plunger) than the more brittle agars which only permitted 4 mm deformation before the break point of the gels was reached. Moreover, the bacterial gel was found to have shorter "setting times" (less than ½ hour) than the "setting times" of the two agars (more than 1 hour), for reaching maximum gel strength after melting and cooling to room temperature. The bacterial gel was stable over a pH range of 2—11 and a sterilisation (20 minutes at 120°C) had no adverse effect on the gel strength.

The Gibco agar is marked by Gibco Europe B.V. in Hoofddorp, Netherlands, and the Oxoid Ion agar No. 2 by Pharmachemie B.V. in Haarlem, Netherlands.

**Claims**

1. Gel-forming polysaccharide built up of repeat units having the formula

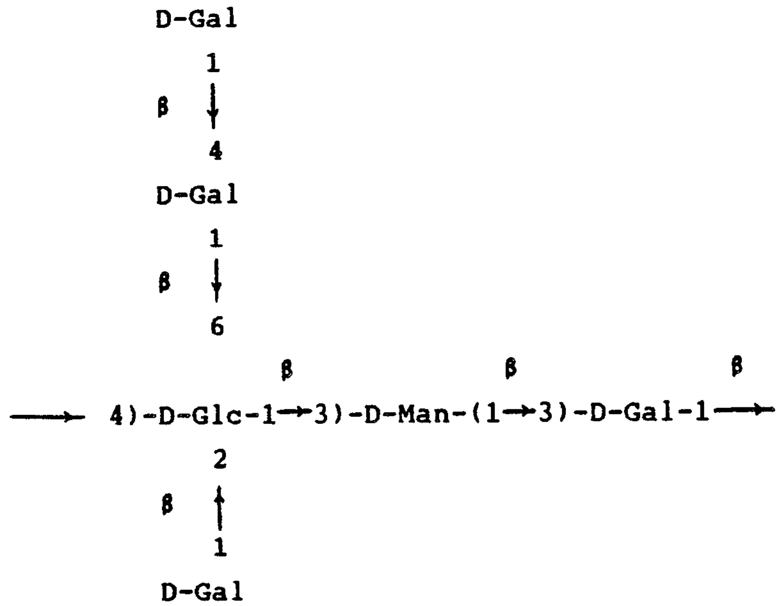

wherein D-Gal, G-Glc and D-Man stand for D-galactose, D-glucose and D-mannose, the polysaccharide having a mean molecular weight of at least 150,000.

2. A process for obtaining the gel-forming polysaccharide defined in claim 1, characterised by extracting cells of the starains *Rhizobium leguminosarum* F—13 or RCR—1044 or the strain *Rhizobium trifolii* TA—1 with the aid of

— either an alkaline solution and subsequently neutralizing the extract obtained with a dilute acid
— or almost boiling water and passing the hot suspension obtained through a bacteriological filter.

3. Process according to claim 2, characterised in that M NaOH is used as the alkaline solution.

4. Process according to claim 3, characterised in that the extraction is carried out at room temperature.

5. Use of the polysaccharide according to claim 1 for forming an aqueous gel.

**Patentansprüche**

1. Gelbildendes Polysaccharid, aufgebaut aus wiederholten Einheiten der Formel

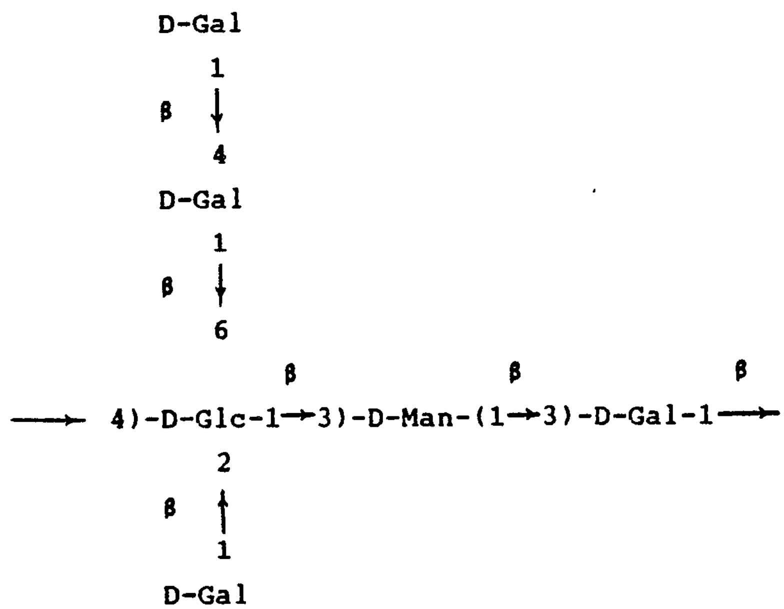

worin D-Gal, D-Glc und D-Man D-Galactose, D-Glucose und D-Mannose bedeuten, welches ein mittleres Molekulargewicht von wenigstens 150 000 hat.

2. Verfahren zur Gewinnung des in Anspruch 1 definierten gelbildenden Polysaccharids, dadurch gekennzeichnet, dass man Zellen der Stämme *Rhizobium leguminosarum* F—13 oder RCR—1044 oder des Stammes *Rhizobium trifolii* TA—1 mit Hilfe von

— entweder einer alkalischen Lösung extrahiert und nachfolgend den erhaltenen Extrakt mit einer verdünnten Säure neutralisiert

— oder fast kochendem Wasser extrahiert und die erhaltene heisse Suspension durch ein bakteriologisches Filter leitet.

3. Verfahren gemäss Anspruch 2, dadurch gekennzeichnet, dass M NaOH als alkalische Lösung verwendet wird.

4. Verfahren gemäss Anspruch 3, dadurch gekennzeichnet, dass die Extraktion bei Raumtemperatur durchgeführt wird.

5. Verwendung des Polysaccharids gemäss Anspruch 1 zur Bildung eines wässrigen Gels.

**Revendications**

1. Polysaccharide formateur de gel constitué de motifs répétitifs ayant la formule

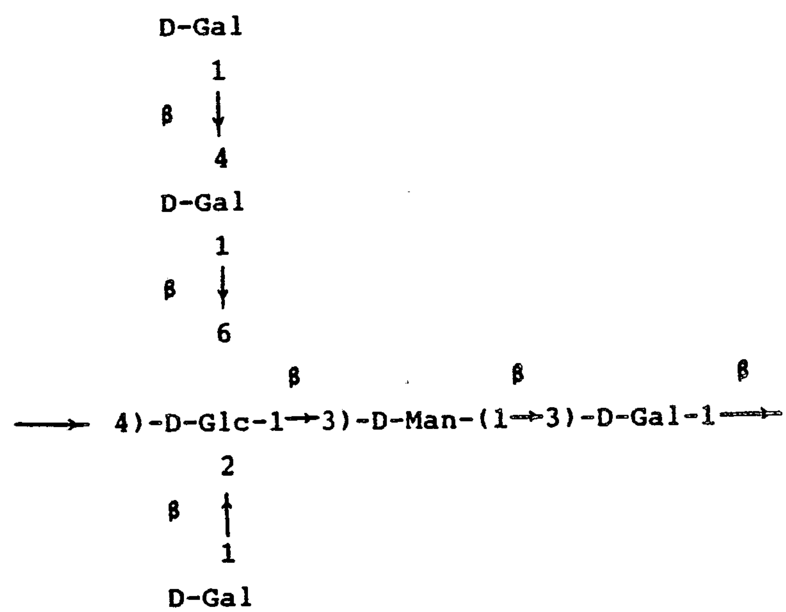

où D-Gal, D-Glc et D-Man représentent le D-galactose, le D-glucose et le D-mannose, le polysaccharide ayant une masse moléculaire moyenne d'au moins 150 000.

2. Procédé pour l'obtention du polysaccharide formateur de gel défini à la revendication 1, caractérisé par l'extraction de cellules de la souche *Rhizobium leguminosarum* F—13 ou RCR—1044 ou de la souche *Rhizobium trifolii* TA—1, à l'aide de

— soit une solution alcaline et ensuite, neutralisation de l'extrait obtenu avec un acide dilué

— soit de l'eau presque bouillante et passage de la suspension chaude obtenue sur un filtre bactériologique.

3. Procédé selon la revendication 2, caractérisé en ce que NaOH M est utilisé comme solution alcaline.

4. Procédé selon la revendication 3, caractérisé en ce que l'extraction est effectuée à la température ambiante.

5. Utilisation du polysaccharide selon la revendication 1, pour former un gel aqueux.

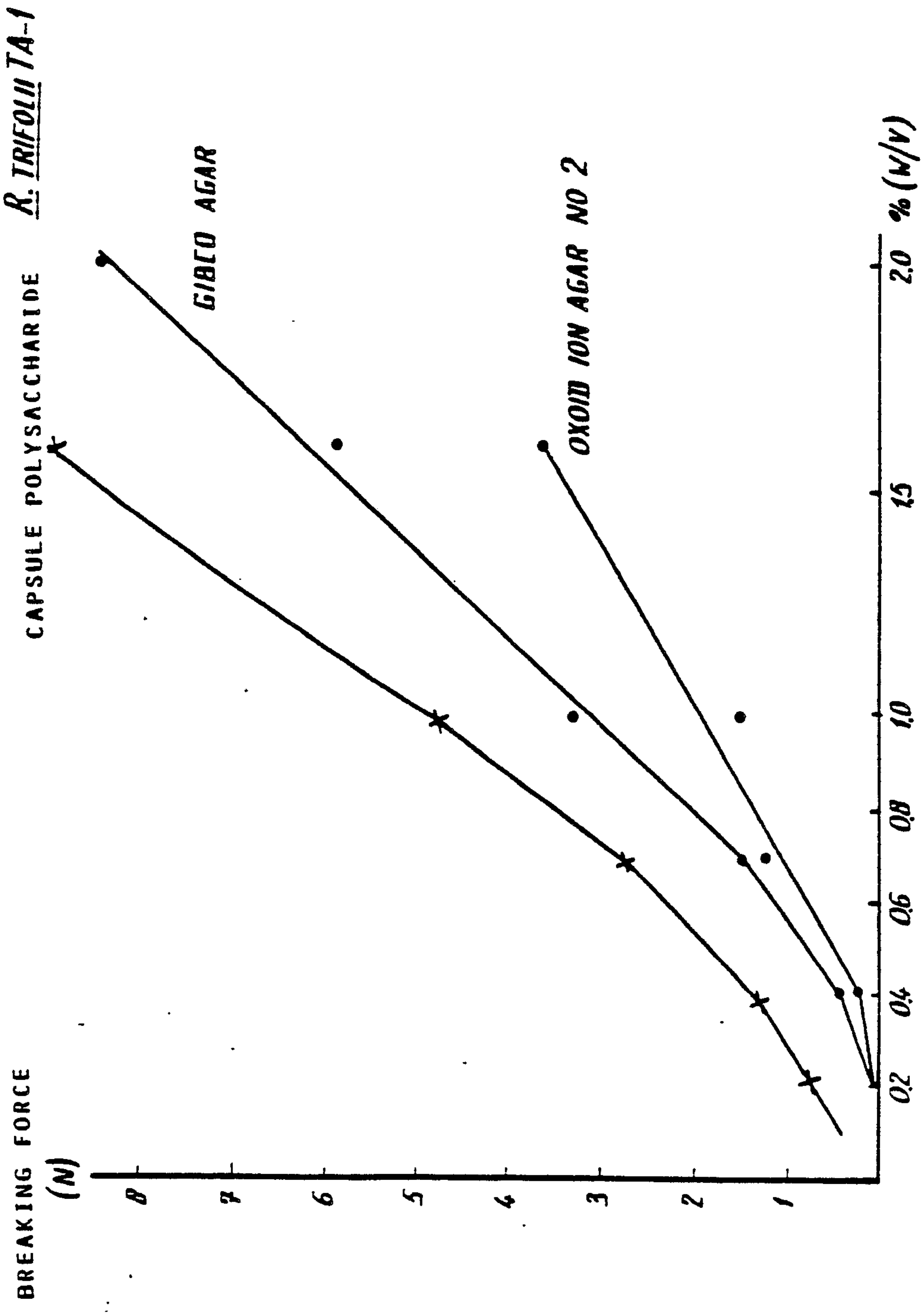
R. TRIFOLII TA-1
BREAKING FORCE (N)
CAPSULE POLYSACCHARIDE
GIBCO AGAR
OXOID ION AGAR NO 2
% (W/V)
1
2
3
4
5
6
7
8
0.2
0.4
0.6
0.8
1.0
1.5
2.0